# EUROPÄISCHE PATENTSCHRIFT

(11) **EP 3 730 106 B1**
(45) Veröffentlichungstag und Bekanntmachung des Hinweises auf die Patenterteilung: **22.09.2021**
(21) Anmeldenummer: 19171418.7
(22) Anmeldetag: 26.04.2019
(51) Int. Cl.: A61F 9/007, A61M 5/32

(54) **VORRICHTUNG ZUM MONTIEREN EINER HOHLNADEL AN EINEM PHACO-INSTRUMENTENHANDSTÜCK UND ZUM DEMONTIEREN VON DIESEM**
DEVICE FOR MOUNTING A HOLLOW NEEDLE ON A PHACO-INSTRUMENT HANDPIECE AND FOR REMOVING SAME
DISPOSITIF DE MONTAGE D'UNE AIGUILLE CREUSE SUR UNE PIÈCE À MAIN D'INSTRUMENT PHACO ET SON DÉMONTAGE

(43) Veröffentlichungstag der Anmeldung: 28.10.2020
(73) Patentinhaber: Wefis GmbH, 50996 Köln (DE)
(72) Erfinder: Wernz, Dietrich, 50996 Köln (DE)
(74) Vertreter: dompatent von Kreisler Selting Werner - Partnerschaft von Patent- und Rechtsanwälten mbB

(56) Entgegenhaltungen:
- WO-A2-2011/149727
- CN-A- 108 992 744
- US-A1- 2009 157 009

## Beschreibung

Die Erfindung betrifft eine Vorrichtung zum Montieren einer Hohlnadel an einem Phaco-Instrumentenhandstück und zum Demontieren der Hohlnadel von dem Phaco-Instrumentenhandstück. Bei diesem chirurgischen Instrument kann es sich beispielsweise um ein Phaco-Emulsifikationshandstück, um ein Phaco-Spülhandstück, um ein Phaco-Saughandstück oder um ein kombiniertes Phaco-Spül/Saughandstück handeln.

In der Ophthalmologie wird für die Behandlung der Augenlinse ein chirurgisches Instrument eingesetzt, das ein Phaco-Emulsifikationshandstück mit einer mit diesem verschraubten Hohlnadel aufweist. Es ist bekannt, die Hohlnadel auf das Handstück mithilfe von drehmomentbegrenzenden manuell handzuhabenden Werkzeugen aufzuschrauben. Als Beispiel für diesen Stand der Technik sei auf WO-A-2011/149727 und US-A-2014/0100515 verwiesen. Die in diesen beiden Schriften beschriebenen drehmomentbegrenzenden Werkzeuge sind konstruktiv recht aufwändig gestaltet.

Aufgabe der Erfindung ist es, eine Vorrichtung zum Montieren einer Hohlnadel an einem Phaco-Instrumentenhandstück und zum Demontieren von diesem anzugeben, die konstruktiv einfach ausgeführt ist und die zum Demontieren der Hohlnadel von dem Phaco-Instrumentenhandstück keine Drehmomentbegrenzung aufweist.

Zur Lösung dieser Aufgabe wird mit der Erfindung eine Vorrichtung zum Montieren einer Hohlnadel an einem Phaco-Instrumentenhandstück und zum Demontieren von diesem vorgeschlagen, die versehen ist mit
- einem manuell handzuhabenden Gehäuse,
- einer in dem Gehäuse drehbar gelagerten Drehwelle, die ein sich aus einer Öffnung des Gehäuses nach außen erstreckendes Aufsteckende zum Aufstecken auf die Hohlnadel aufweist, wobei das Aufsteckende innenliegende Zusammengreifvorsprünge zum Ineinandergreifen mit Ausnehmungen in der Außenseite der Hohlnadel zwecks Mitdrehens der Hohlnadel beim manuellen Drehen des Gehäuses aufweist,
- einer Drehmomentbegrenzungs- und Rücklaufsperrkupplung, die eine an der Drehwelle angeordnete Anlageflächen als eine erste Kupplungsfläche aufweist, und
- einem koaxial zur Drehwelle im Gehäuse angeordneten Mitnehmeelement, das mit dem Gehäuse gekoppelt ist und eine Radialanlagefläche als zweite Kupplungsfläche der Drehmomentbegrenzung-und Rücklaufsperrkupplung aufweist,
- wobei die beiden Kupplungsflächen federnd gegeneinander vorgespannt aneinander anliegen,
- wobei die eine Kupplungsfläche mindestens einen Kupplungsfreigabevorsprung und die andere Kupplungsfläche mindestens eine zur Form des Kupplungsfreigabevorsprungs komplementäre Kupplungsvertiefung aufweist,
- wobei die eine Kupplungsfläche mindestens einen Rücklaufsperrvorsprung und die andere Kupplungsfläche mindestens eine Rücklaufsperrvertiefung aufweist,
- wobei die mindestens eine Rücklaufsperrvertiefung - in Umfangsrichtung einer zur Achse der Drehwelle konzentrischen Kreislinie betrachtet, - eine Erstreckung aufweist, die größer ist, insbesondere mehr als das doppelte größer ist, als die Erstreckung des Rücklaufsperrvorsprungs in Umfangsrichtung der Kreislinie,
- wobei der mindestens eine Rücklaufsperrvorsprung an einem der beiden in Umfangsrichtung der Kreislinie betrachtet angeordneten Enden der Rücklaufsperrvertiefung anliegt, wenn der mindestens eine Kupplungsfreigabevorsprung in die mindestens eine Kupplungsvertiefung eingetaucht ist,
- wobei der mindestens eine Rücklaufsperrvorsprung - in axialer Erstreckung der Drehwelle betrachtet - eine Höhe aufweist, die geringer ist als die Höhe des mindestens einen Kupplungsfreigabevorsprungs, und
- wobei der mindestens eine Rücklaufsperrvorsprung vollständig aus der mindestens einen Rücklaufsperrvertiefung herausbewegt ist, wenn der mindestens eine Kupplungsfreigabevorsprung aus der mindestens einen Kupplungsvertiefung herausbewegt ist und sich oberhalb der oder auf der die Rücklaufsperrvertiefung aufweisenden Kupplungsfläche bewegt, bis der mindestens eine Kupplungsfreigabevorsprung beim Weiterdrehen des Gehäuses in die mindestens eine Kupplungsvertiefung wieder eingetaucht ist.

Mit der Erfindung wird also eine Vorrichtung vorgeschlagen, die ein manuell handzuhabendes, mit den Fingern einer Hand zu ergreifendes Gehäuse aufweist, in dem eine Drehwelle drehbar gelagert ist. In dem Gehäuse ist ferner ein Mitnehmerelement angeordnet, das Teil des Gehäuses oder aber auch ein von dem Gehäuse separates Element sein kann. Das Mitnehmerelement dreht sich mit dem Gehäuse, wenn dieses per Hand gedreht wird. Zwischen dem Mitnehmerelement und der Drehwelle befindet sich eine Drehmomentbegrenzungs- und Rücklaufsperrkupplung, die zwei federnd gegeneinander gedrückte Kupplungsflächen aufweist. Eine dieser Kupplungsflächen befindet sich an der Drehwelle, während sich die andere Kupplungsfläche am Mitnehmerelement befindet. Typischerweise sind beide Kupplungsflächen als sich radial zur Drehwelle erstreckende radiale Anlageflächen ausgeführt.

Die Drehwelle weist darüber hinaus ein aus einer Öffnung des Gehäuses herausgeführtes Aufsteckende auf, das eine Aufnahmevertiefung bildet, die auf die Hohlnadel aufgesteckt ist, wenn die Hohlnadel montiert oder demontiert werden soll. Die Hohlnadel ist typischerweise an ihrer Außenseite mit Ausnehmungen versehen, in die Zusammengreifvorsprünge an der Innenseite der Aufnahmevertiefung des Aufsteckendes eintauchen. Alternativ kann die Hohlnadel an ihrer Außenseite Zusammengreifvorsprünge zur Aufnahme in Vertiefungen an der Innenseite der Aufnahmevertiefung des Aufsteckendes aufweisen. In beiden Fällen entsteht eine Art Formschluss, wodurch die Hohlnadel mitgedreht wird, wenn das Gehäuse manuell gedreht wird.

Die beiden Kupplungsflächen sind mit bezüglich ihrer Form komplementären Kupplungsfreigabevorsprünge und Kupplungsvertiefungen versehen. Dabei können die Vorsprünge sämtlich auf der einen Kupplungsfläche und die komplementären Kupplungsvertiefungen sämtlich auf der anderen Kupplungsfläche angeordnet sein. Es ist aber auch möglich, dass Vorsprünge und Vertiefungen auf beiden Kupplungsflächen ausgebildet sind. Nach der Erfindung bedarf es mindestens eines Kupplungsfreigabevorsprungs und mindestens einer Kupplungsvertiefung. Durch die federnde Vorspannung der beiden Kupplungsflächen liegen diese solange aneinander an und sind die Kupplungsfreigabevorsprünge solange in die ihnen zugeordneten Kupplungsvertiefungen eingetaucht, bis das Drehmoment einen Grenzwert überschreitet. Die Größe dieses Werts ist konstruktiv bedingt, und zwar beispielsweise einerseits durch die Kraft, mit der die beiden Kupplungsflächen aneinander liegen und anderseits durch die Ausgestaltung (d. h. Geometrie) der Kupplungsfreigabevorsprünge und Kupplungsvertiefungen. Typischerweise sind die Kupplungsfreigabevorsprünge und die Kupplungsvertiefungen sphärisch ausgebildet (beispielsweise als Halbkugel bzw. Halbschale).

Damit nun die Drehmomentbegrenzung nur beim Aufschrauben der Hohlnadel an dem Phaco-Instrumentenhandstück wirkt (nachfolgend wird hier repräsentativ für derartige Instrumente von einem Phaco-Emulsifikationshandstück gesprochen), ist bei der erfindungsgemäßen Vorrichtung eine Rücklaufsperre vorgesehen. Diese Rücklaufsperre weist Rücklaufsperrvorsprünge und Rücklaufsperrvertiefungen an den Kupplungsflächen auf, wobei auch hier gilt, dass sämtliche Rücklaufsperrvorsprünge an der einen Kupplungsfläche und sämtliche Rücklaufsperrvertiefungen an der anderen Kupplungsfläche angeordnet sind oder aber dass beide Kupplungsflächen sowohl Rücklaufsperrvorsprünge als auch Rücklaufsperrvertiefungen aufweisen. Nach der Erfindung bedarf es mindestens eines Rücklaufsperrvorsprungs und mindestens einer Rücklaufsperrvertiefung.

Die relative Anordnung der Rücklaufsperrvorsprünge und der Rücklaufsperrvertiefungen ist erfindungsgemäß derart gewählt, dass sich jeder Rücklaufsperrvorsprung in der ihm zugeordneten Rücklaufsperrvertiefung in der einen Drehrichtung des Gehäuses um die Achse der Drehwelle verschieben kann, wobei es sich bei dieser Drehrichtung um diejenige handelt, in der die Drehmomentbegrenzung wirkt. In dieser Drehrichtung bewegen sich dann die Kupplungsfreigabevorsprünge bei Erreichen des maximal zulässigen Drehmoments aus ihren Kupplungsvertiefungen heraus, wodurch beide Kupplungsflächen um die Höhe der Kupplungsfreigabevorsprünge beabstandet sind. In diesem Zustand sind auch die Rücklaufsperrvorsprünge an ihren Rücklaufsperrvertiefungen heraus bewegt. Erfindungsgemäß sind nun die Rücklaufsperrvorsprünge flacher als die Kupplungsfreigabevorsprünge, weisen also in axialer Erstreckung der Drehwelle betrachtet eine geringere Höhe als die Kupplungsfreigabevorsprünge auf. Jede Rücklaufsperrvertiefung weist zwei in Drehrichtung des Gehäuses und entgegengesetzt dazu liegende Enden bzw. Endanschläge auf. Bevor ein Rücklaufsperrvorsprung, der in die ihm zugeordnete Rücklaufsperrvertiefung eingetaucht ist, das in derjenigen Drehrichtung liegende Ende erreicht, in der die Drehmomentbegrenzung wirkt, ist er bei Überschreitung der Drehmomentsbegrenzung bereits aus der Rücklaufsperrvertiefung herausgehoben, da nämlich die Kupplungsfreigabevorsprünge aus den Kupplungsvertiefungen herausbewegt sind. Dagegen liegt jeder Rücklaufsperrvorsprung in der Rücklaufsperrvertiefung bereits an dessen anderen Ende an, wenn die Kupplungsfreigabevorsprünge in den Kupplungsvertiefungen positioniert sind. Damit lassen es die Rücklaufsperrvorsprünge in entgegengesetzter Drehrichtung nicht zu, dass sich die Kupplungsfreigabevorsprünge aus den Kupplungsvertiefungen heraus bewegen können, womit die Drehmomentsbegrenzung in dieser entgegengesetzten Drehrichtung aufgehoben und damit inaktiv ist.

Die Kupplungsfreigabevorsprünge und die Kupplungsvertiefungen können auf Kreislinien angeordnet sein, die konzentrisch zur Achse der Drehwelle verlaufen und miteinander fluchten, also den gleichen Radius aufweisen. Ebenso können die Rücklaufsperrvorsprünge und die Rücklaufsperrvertiefungen auf Kreislinien angeordnet sein, die miteinander fluchten. Typischerweise sind aber die Kreislinien der Kupplungsfreigabevorsprünge und -vertiefungen andere als diejenigen der Rücklaufsperrvorsprünge und -vertiefungen, was aber nicht zwingend so sein muss.

In weiterer zweckmäßiger Ausgestaltung der Erfindung kann vorgesehen sein, dass die eine Kupplungsfläche mehrere Kupplungsfreigabevorsprünge und die andere Kupplungsfläche eine der Anzahl der Kupplungsfreigabevorsprünge gleichende Anzahl von Kupplungsvertiefungen aufweist oder eine Anzahl von Kupplungsvertiefungen aufweist, die um den Faktor 2 oder 3 oder mehr größer ist als die Anzahl der Kupplungsfreigabevorsprünge, wobei die relative Anordnung einer jeweils der Anzahl der Kupplungsfreigabevorsprünge gleichenden Anzahl von Kupplungsvertiefungen gleich der relativen Anordnung der Kupplungsfreigabevorsprünge ist.

In weiterer zweckmäßiger Ausgestaltung der Erfindung kann vorgesehen sein, dass die eine Kupplungsfläche eine größere Anzahl von Rücklaufsperrvertiefungen aufweist, als Rücklaufsperrvorsprünge an der anderen Kupplungsfläche angeordnet sind.

Zweckmäßig kann es ferner sein, wenn die Anzahl der Rücklaufsperrvertiefungen gleich oder um den Faktor 2 oder 3 oder mehr größer ist als die Anzahl der Rücklaufsperrvorsprünge.

Ferner kann es von Vorteil sein, wenn die eine Kupplungsfläche drei um jeweils 120° zueinander versetzt angeordnete Kupplungsfreigabevorsprünge und die andere Kupplungsfläche sechs um jeweils 60° zueinander versetzt angeordnete Kupplungsvertiefungen aufweist und wenn die eine Kupplungsfläche drei um jeweils 120° zueinander versetzt angeordnete Rücklaufsperrvorsprünge und die andere Kupplungsfläche sechs um jeweils 60° zueinander versetzt angeordnete Rücklaufsperrvertiefungen aufweist, wobei die Rücklaufsperrvertiefungen jeweils mit der Mitte zwischen zwei Kupplungsvertiefungen fluchtend angeordnet sind.

In weiterer zweckmäßiger Ausgestaltung der Erfindung kann vorgesehen sein, dass der mindestens eine oder jeder Kupplungsfreigabevorsprung und die mindestens eine oder jede Kupplungsfreigabevertiefung auf einer konzentrisch zur Drehwelle verlaufenden ersten Kreislinie angeordnet sind und dass der mindestens eine oder jeder Rücklaufsperrvorsprung und die mindestens eine oder jede Rücklaufsperrvertiefung auf einer konzentrisch zur Drehwelle verlaufenden zweiten Kreislinie angeordnet ist, die verschieden ist von der ersten Kreislinie. Es ist aber auch möglich, dass sich Kupplungsfreigabevertiefungen und Kupplungsfreigabevorsprünge längs mehrerer erster Kreislinien verteilen und dementsprechend auch Rücklaufsperrvorsprünge und Rücklaufsperrvertiefungen längs mehrerer zweiter Kreislinien verteilen.

In weiterer zweckmäßiger Ausgestaltung der Erfindung ist vorgesehen, dass die Drehwelle in dem Gehäuse mittels Lagerbuchsen drehbar gelagert ist und/oder dass die Drehwelle sich durch das Mitnehmerelement hindurch erstreckt und/oder dass auf der Drehwelle eine Schraubendruckfeder konzentrisch angeordnet ist, die sich zwischen einer Stützfläche im Gehäuse und einer Stützfläche an der Drehwelle erstreckt und abstützt und/oder dass das Mitnehmerelement mittels einer Schraubendruckfeder gegen die Drehwelle gedrückt ist.

Schließlich ist es auch denkbar, dass der mindestens eine oder jeder Zusammengreifvorsprung und/oder der mindestens eine oder jeder Kupplungsfreigabevorsprung sphärisch ausgebildet ist und/oder dass der mindestens eine oder jeder Rücklaufsperrvorsprung und die mindestens eine oder jede Rücklaufsperrvertiefung rechtwinklig oder im Wesentlichen rechtwinklig zu den Kupplungsflächen verlaufende Anlageflächen aufweisen, die aneinander liegen, wenn der mindestens eine Kupplungsfreigabevorsprung in die mindestens eine Kupplungsvertiefung eingetaucht ist.

Die erfindungsgemäße Vorrichtung ist zweckmäßigerweise vollständig oder ganz überwiegend aus Kunststoffmaterial gefertigt (beispielsweise in Form von Kunststoff-Spritzgussteilen), was insbesondere für die Kupplung gilt. Je mehr Rücklaufsperrvorsprünge vorgesehen sind, umso scherfester liegen die Kupplungsflächen aneinander, wenn es um das Abschrauben der Hohlnadel geht.

Die Erfindung wird nachfolgend anhand eines Ausführungsbeispiels sowie unter Bezugnahme auf die Zeichnung näher erläutert. Im Einzelnen zeigen dabei:
- Fig. 1: eine perspektivische Ansicht eines Ausführungsbeispiels der erfin-dungsgemäßen Vorrichtung zum Montieren und Demontieren einer Hohlnadel an einem Phaco-Emulsifikationshandstück und von diesem,
- Fig. 2: in Seitenansicht die Vorrichtung nach Fig. 1 mit Andeutung ihres Auf-schiebens auf eine Hohlnadel zur Montage an einem Phaco-Emulsifikationshandstück,
- Fig. 3: perspektivisch und in Explosionsdarstellung den Aufbau der Vorrich-tung nach Fig.1,
- Fig. 4: eine perspektivische Ansicht auf die Kupplungsflächen der Drehmo-mentbegrenzungs- und Rücklaufsperrkupplung der Vorrichtung nach Fig. 1,
- Fign. 5 und 6: Seitenansichten und Querschnittsansichten der Kupplung in zwei ver-schiedenen Verdrehpositionen der beiden Kupplungsteile,
- Fig. 7: eine Seitenansicht der Kupplung bei Eingriff ihrer Kupplungsflächen und
- Fign. 8 und 9: Schnittansichten entsprechend VIII-IX der Fig. 7.

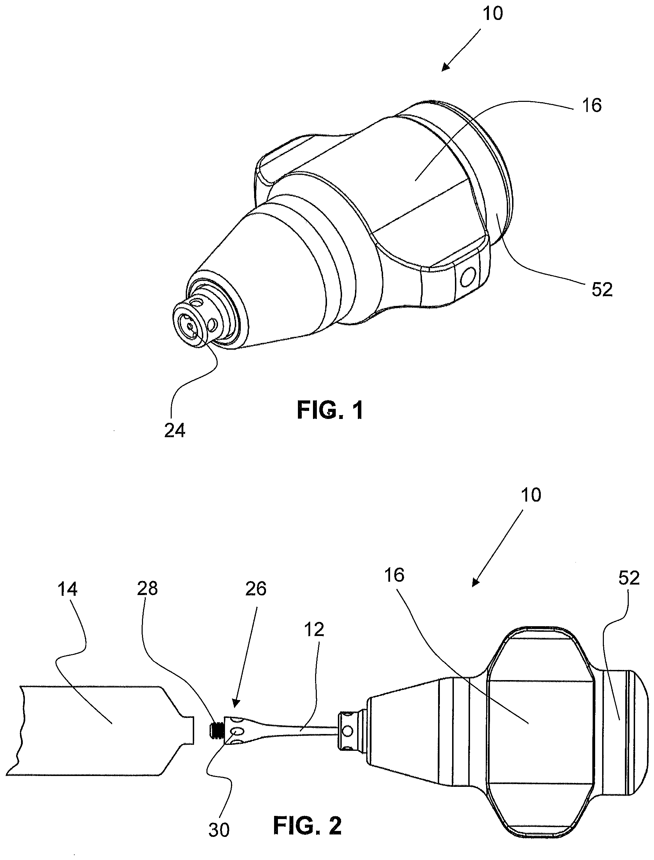

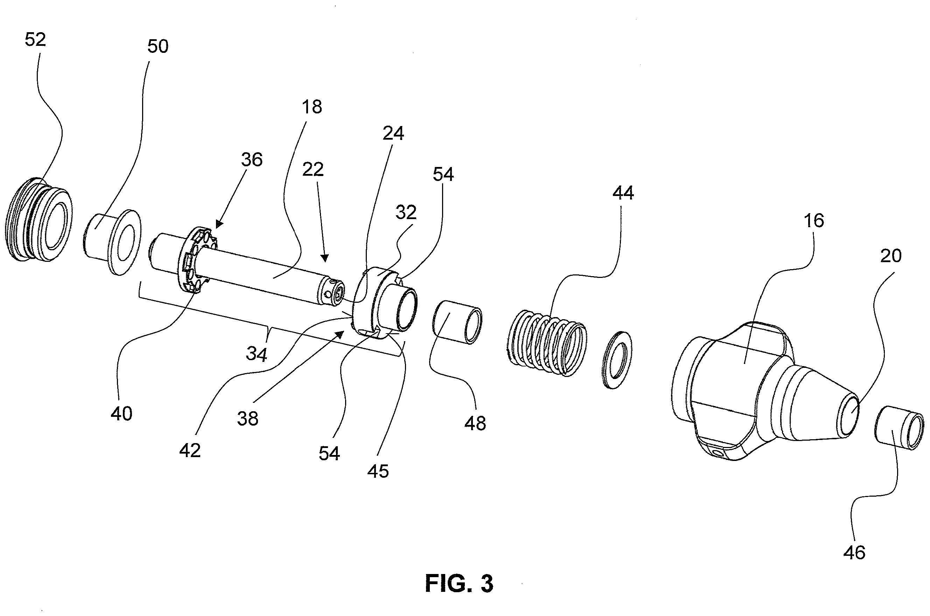

In den Fign. 1 bis 3 sind verschiedene Ansichten eines Ausführungsbeispiels einer Vorrichtung 10 zum Montieren einer Hohlnadel 12 an einem Phaco-Emulsifikationshandstück 14 und zum Demontieren der Hohlnadel 12 von dem Phaco-Emulsifikationshandstück 14 gezeigt. Die Vorrichtung 10 weist ein mit den Fingern einer Hand zu ergreifendes Gehäuse 16 auf, in dem eine Drehwelle 18 drehbar gelagert ist, die sich durch eine Öffnung (20) des Gehäuses 16 hindurch nach außen erstreckt. Dieses Ende der Drehwelle 18 ist das Aufsteckende 22, das hohl ausgebildet ist und innenliegende Zusammengreifvorsprünge 24 aufweist, die unter anderem in den Fign. 1 und 2 angedeutet sind. Die Hohlnadel 12 weist einen konischen Abschnitt 26 mit sich daran anschließendem Gewindestutzen 28 auf. Innerhalb des konischen Abschnittes 26 weist die Hohlnadel 12 an ihrer Außenseite Ausnehmungen 30 auf, in die die Zusammengreifvorsprünge 24 eingreifen, wenn sich die Hohlnadel 12 vollständig in dem Gehäuse 16 befindet.

Die Drehwelle 18 bildet zusammen mit einem Mitnehmerelement 32 eine Drehmomentbegrenzungs- und Rücklaufsperrkupplung 34 (nachfolgend der Einfachheit halber Kupplung genannt), die eine erste Kupplungsfläche 36 und eine zweite Kupplungsfläche 38 aufweist. Die erste Kupplungsfläche 36 ist als eine in diesem Ausführungsbeispiel radiale Anlagefläche 40 an der Drehwelle 18 ausgebildet, während die zweite Kupplungsfläche 38 als radiale Anlagefläche 42 des Mitnehmerelements 32 ausgebildet ist. Beide Kupplungsflächen 36, 38 sind federelastisch gegeneinander gespannt, und zwar mittels einer Feder 44, die in diesem Ausführungsbeispiel als Spiraldruckfeder ausgebildet ist. Über Lagerhülsen 46, 48, 50 ist die Drehwelle 18 innerhalb des Gehäuses 16 drehbar gelagert. Die Drehwelle 18 durchdringt dabei das Mitnehmerelement 32. Auf der der ersten Kupplungsfläche 36 abgewandten Seite des Mitnehmerelements 32 befindet sich die koaxial auf der Drehwelle 18 sitzende Feder 44. An dem der Öffnung 20 für das Aufsteckende 22 der Drehwelle 18 abgewandten Ende des Gehäuses 16 befindet sich ein Verschlussdeckel 52. Die Feder 44 stützt sich einerseits an einer Stützfläche im Gehäuse 16 (nicht gezeigt) und andererseits an einer Stützfläche 45 ab, die an dem Mitnehmerelement 32 angeordnet ist, und zwar der zweiten Kupplungsfläche 38 des Mitnehmerelements 32 abgewandt.

Das Mitnehmerelement 32 ist formschlüssig in dem Gehäuse 16 angeordnet. Hierzu weist das Mitnehmerelement 32 zwei Umfangsvertiefungen 54 auf, die mit innenliegenden Mitnehmernasen (nicht dargestellt) in dem Gehäuse 16 zusammengreifen. Bei einer Verdrehung des Gehäuses 16 wird also in diesem das Mitnehmerelement 32 mitgedreht, wobei es sich axial auf der Drehwelle 18 verschieben kann, ohne dass die Mitnehmernasen des Gehäuses 16 außer Eingriff aus den Umfangsvertiefungen 54 geraten.

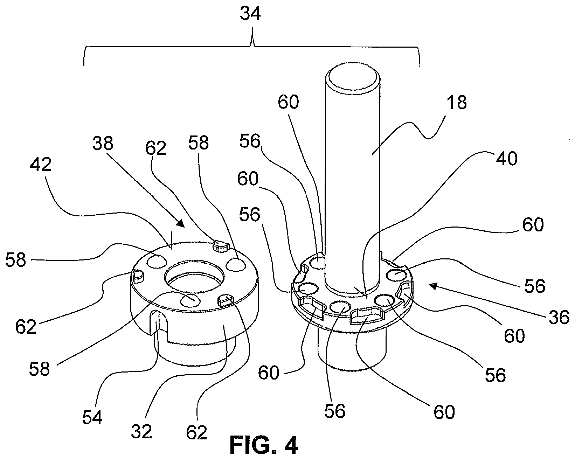

Wesentliches Element der Vorrichtung 10 ist die Kupplung 34, die in Fig. 4 perspektivisch nochmals dargestellt ist. Die Kupplung 34 wird letztendlich von der Drehwelle 18 und dem Mitnehmerelement 32 gebildet. Die erste Kupplungsfläche 36 weist in diesem Ausführungsbeispiel sechs Kupplungsvertiefungen 56 auf, die wahlweise mit drei Kupplungsvorsprüngen 58 der zweiten Kupplungsfläche 38 zusammenwirken. Die erste Kupplungsfläche 36 weist darüber hinaus in diesem Ausführungsbeispiel sechs Rücklaufsperrvertiefungen 60 auf, die mit drei Rücklaufsperrvorsprüngen 62 der zweiten Kupplungsfläche 36 zusammenwirken.

Die erfindungsgemäße Kupplung 34 dient dazu, in der einen von zwei entgegengesetzten Drehbewegungen des Gehäuses 16 für eine Drehmomentbegrenzung zu sorgen. Bei dieser Drehbewegung handelt es sich um diejenige Drehbewegung, um die das Gehäuse gedreht werden muss, um die Hohlnadel 12 aufzuschrauben. Die weitere Funktion der Kupplung 34 besteht darin, in entgegengesetzter Drehrichtung des Gehäuses 16 eine Drehmomentbegrenzung auszuschließen. Diese nichtgegebene Drehmomentbegrenzung wird nachfolgend auch "Rücklaufsperre" genannt, was bedeutet, dass die Drehmomentbegrenzung gesperrt, das heißt aufgehoben ist.

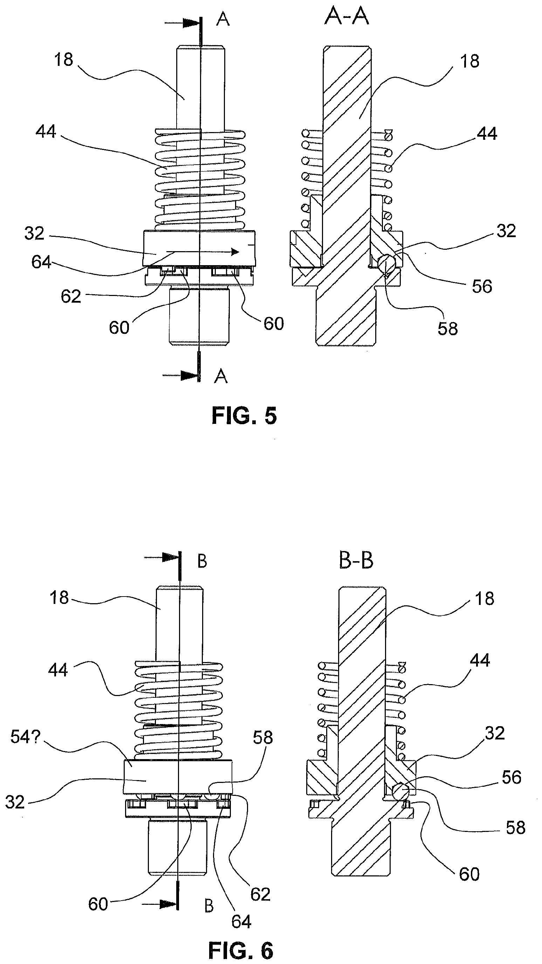

Fig. 5 zeigt die Situation, in der die drei Kupplungsfreigabevorsprünge 58 in drei der sechs Kupplungsvertiefungen 56 eingetaucht ist. In diesem Zustand sind auch die drei Rücklaufsperrvorsprünge 62 in drei der sechs Rücklaufsperrvertiefungen 60 eingetaucht. Das Mitnehmerelement 32 ist drehfest im Gehäuse 16 angeordnet. Wird nun das Gehäuse 16 gedreht, so dass sich das Mitnehmerelement 32 in Richtung des Pfeils 64 (siehe Fig. 5) mitdreht, so nimmt das Mitnehmerelement 32 solange die Drehwelle 18 mit, wie das durch die Stärke der Feder 44 und durch die Anpressung der beiden Kupplungsflächen sowie durch die Ausgestaltung der Kupplungsfreigabevorsprünge 58 und der Kupplungsvertiefungen 56 gegebene Drehmoment nicht überschritten wird. Sobald dies erfolgt, rücken die Kupplungsfreigabevorsprünge 58 aus den Kupplungsvertiefungen 56 heraus, womit sich das Mitnehmerelement 32 gegen die Kraft der Feder 44 innerhalb des Gehäuses 16 axial verschiebt, wie es in Fig. 6 gezeigt ist. Damit dies in der besagten Aufschraub-Drehbewegung des Gehäuses 16 funktioniert, müssen sich die Rücklaufsperrvorsprünge 62 ebenfalls aus den Rücklaufsperrvertiefungen 60 heraus bewegen können. Dies wiederum gelingt dadurch, dass die Rücklaufsperrvertiefungen 60 in Umfangsrichtung der Drehwelle 18 betrachtet eine größere Erstreckung aufweisen als die Rücklaufsperrvorsprünge 62. Dies ist beispielsweise in Fig. 5 gezeigt. Die Rücklaufsperrvertiefungen 60 sind etwa doppelt so breit (in Umfangsrichtung der Drehwelle 18 betrachtet) wie die Rücklaufsperrvorsprünge 62.

In dem Zustand des Kupplungseingriffs (siehe beispielsweise Fig. 5) liegen die Rücklaufsperrvorsprünge 62 an dem einen Ende der Rücklaufsperrvertiefungen 60 an und sind zum anderen Ende der Rücklaufsperrvertiefungen 60 frei. Diese freie Strecke reicht nun aus, damit sich bei Erreichen der Drehmomentbegrenzung das Mitnehmerelement 32 relativ zur Drehwelle 18 weiterdrehen kann, wodurch sich die Kupplungsfreigabevorsprünge 58 aus den Kupplungsfreigabevertiefungen 56 herausbewegen können, mit der Folge, dass die Rücklaufsperrvorsprünge 62 sich jetzt oberhalb der ersten Kupplungsfläche 36 befinden und damit das Mitnehmerelement 32 sich solange weiterdrehen kann, bis die Kupplungsfreigabevorsprünge 58 in die in Drehrichtung nächstgelegenen Kupplungsfreigabevertiefungen 56 wieder eintauchen. Dann tauchen auch die Rücklaufsperrvorsprünge 62 in den nächstgelegenen Rücklaufsperrvertiefungen 60 wieder ein. Dieser Prozess des "Kupplungsrutschens" setzt sich dann bei weiterer Verdrehung des Gehäuses 16 fort, was dem Benutzer signalisiert (ggf. auch durch eine entsprechende Akustik), dass das maximal zulässige Drehmoment zum Aufschrauben der Hohlnadel 12 auf das Phaco-Emulsifikationshandstück 14 erreicht ist.

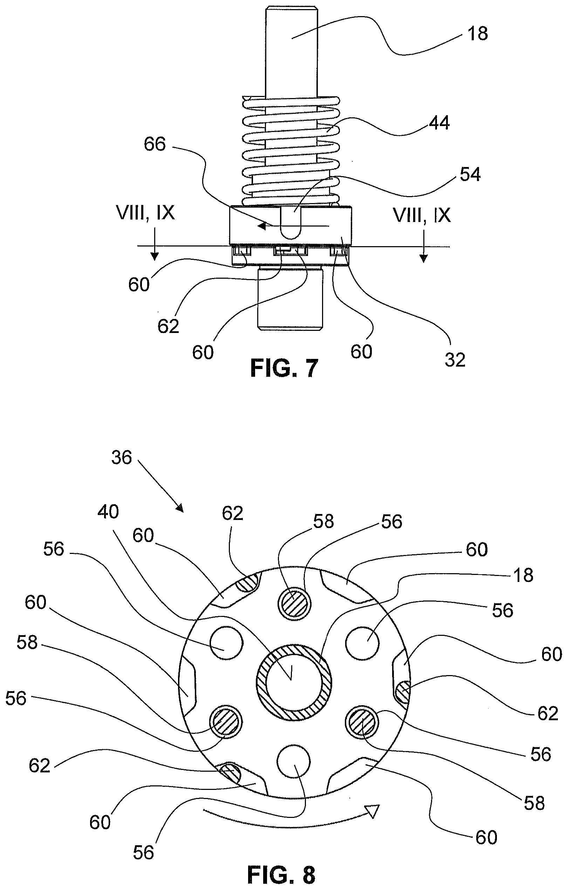

Im umgekehrten Fall, also beim Verdrehen des Gehäuses 16 zum Abschrauben der Hohlnadel 12 ergibt sich wiederum als Ausgangssituation die Situation gemäß Fig. 5 bzw. Fig. 7. Wird nun das Gehäuse 16 dergestalt verdreht, dass sich das Mitnehmerelement 32 in Richtung des Pfeils 66 dreht, blockieren die Rücklaufsperrvorsprünge 62 eine Relativverdrehung von Mitnehmerelement 32 und Drehwelle 18, da sie in Drehrichtung des Pfeils 66 betrachtet direkt an den in dieser Drehrichtung 66 liegenden Rändern der Rücklaufsperrvertiefungen 60 anliegen.

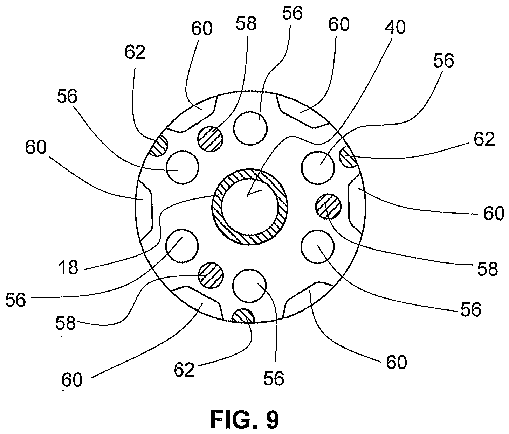

Die Fign. 8 und 9 verdeutlichen die zuvor beschriebene Funktionen nochmals. Ausgangspunkt ist Fig. 8, die die Kupplung 34 im Eingriff zeigt. Die drei Kupplungsfreigabevorsprünge 58 befinden sich in drei der sechs Kupplungsvertiefungen 56, während die drei Rücklaufsperrvorsprünge 62 in drei der sechs Rücklaufsperrvertiefungen 60 eingetaucht sind, und zwar, bezogen auf Fig. 8, an deren in Uhrzeigerrichtung betrachteten Begrenzungen. Wird nun, wie in Fig. 9 gezeigt, das Gehäuse 16 zum Aufschrauben der Hohlnadel 12 verdreht (siehe den Pfeil 64), so treten die Kupplungsfreigabevorsprünge 58 bei Überschreiten des maximalen Drehmoments aus den Kupplungsvertiefungen 56 heraus. Da die Rücklaufsperrvorsprünge 62 in axialer Richtung betrachtet eine geringere Höhe aufweisen als die Kupplungsfreigabevorsprünge 58, bewegen sich die Rücklaufsperrvorsprünge 62 oberhalb der betreffenden Kupplungsfläche 36, lassen also die Drehmomentbegrenzung zu. In umgekehrter Drehrichtung, also entgegengesetzt zum Pfeil 64 der Fig. 9 sperren die Rücklaufsperrvorsprünge 62 die Drehmomentbegrenzung und heben diese damit auf. Das Mitnehmerelement 32 wird also die Drehwelle 18 in jedem Fall mitnehmen, was zum Abschrauben der Hohlnadel 12 von dem Phaco-Emulsifikationshandstück 14 von Vorteil ist.

### BEZUGSZEICHENLISTE

- 10: Vorrichtung
- 12: Hohlnadel
- 14: Phaco-Emulsifikationshandstück
- 16: Gehäuse
- 18: Drehwelle des Gehäuses
- 20: Öffnung des Gehäuses
- 22: Aufsteckende der Drehwelle
- 24: Zusammengreifvorsprünge
- 26: konischer Abschnitt der Hohlnadel
- 28: Gewindestutzen der Hohlnadel
- 30: Ausnehmungen im Abschnitt 26
- 32: Mitnehmerelement des Gehäuses
- 34: Drehmomentbegrenzungs- und Rücklaufsperrkupplung
- 36: erste Kupplungsfläche
- 38: zweite Kupplungsfläche
- 40: erste Anlagefläche
- 42: zweite Anlagefläche
- 44: Feder
- 45: Stützfläche an dem Mitnehmerelement für die Feder
- 46: Lagerhülse
- 48: Lagerhülse
- 50: Lagerhülse
- 52: Verschlussdeckel des Gehäuses
- 54: Umfangsvertiefungen am Mitnehmerelement
- 56: Kupplungsvertiefungen
- 58: Kupplungsfreigabevorsprung
- 60: Rücklaufsperrvertiefungen
- 62: Rücklaufsperrvorsprünge
- 64: Pfeil (Drehrichtung)
- 66: Pfeil (Drehrichtung)

## Patentansprüche

1. Vorrichtung zum Montieren und Demontieren einer Hohlnadel an einem Phaco-Instrumentenhandstück und von diesem, mit
- einem manuell handzuhabenden Gehäuse (16),
- einer in dem Gehäuse (16) drehbar gelagerten Drehwelle (18), die ein sich aus einer Öffnung (20) des Gehäuses (16) nach außen erstreckendes Aufsteckende (22) zum Aufstecken auf die Hohlnadel (12) aufweist, wobei das Aufsteckende (22) innenliegende Zusammengreifvorsprünge (24) zum Ineinandergreifen mit Ausnehmungen (30) in der Außenseite der Hohlnadel zwecks Mitdrehens der Hohlnadel (12) beim manuellen Drehen des Gehäuses (16) aufweist,
- einer Drehmomentsbegrenzungs- und Rücklaufsperrkupplung (34), die eine an der Drehwelle (18) angeordnete Anlagefläche (40) als eine erste Kupplungsfläche (36) aufweist, und
- einem koaxial zur Drehwelle (18) im Gehäuse (16) angeordneten Mitnehmeelement (32), das mit dem Gehäuse (16) gekoppelt ist und eine Anlagefläche (42) als zweite Kupplungsfläche (38) der Drehmomentbegrenzungs- und Rücklaufsperrkupplung (34) aufweist,
**dadurch gekennzeichnet, dass**
- die beiden Kupplungsflächen (36, 38) federnd gegeneinander vorgespannt aneinander anliegen,
- die eine Kupplungsfläche (38) mindestens einen Kupplungsfreigabevorsprung (58) und die andere Kupplungsfläche (36) mindestens eine zur Form des Kupplungsfreigabevorsprungs (58) komplementäre Kupplungsvertiefung (56) aufweist,
- die eine Kupplungsfläche (38) mindestens einen Rücklaufsperrvorsprung (62) und die andere Kupplungsfläche (38) mindestens eine Rücklaufsperrvertiefung (60) aufweist,
- die mindestens eine Rücklaufsperrvertiefung (60) - in Umfangsrichtung einer zur Achse der Drehwelle (18) konzentrischen Kreislinie betrachtet - eine Erstreckung aufweist, die größer ist, insbesondere mehr als das doppelte größer ist, als die Erstreckung des Rücklaufsperrvorsprungs (62) in Umfangsrichtung der Kreislinie,
- der mindestens eine Rücklaufsperrvorsprung (62) an einem der beiden in Umfangsrichtung der Kreislinie betrachtet angeordneten Enden der Rücklaufsperrvertiefung (60) anliegt, wenn der mindestens eine Kupplungsfreigabevorsprung (58) in die mindestens eine Kupplungsvertiefung (56) eingetaucht ist,
- der mindestens eine Rücklaufsperrvorsprung (62) in axialer Erstreckung der Drehwelle (18) betrachtet eine Höhe aufweist, die geringer ist als die Höhe des mindestens einen Kupplungsfreigabevorsprungs (58), und
- der mindestens eine Rücklaufsperrvorsprung (62) vollständig aus der mindestens einen Rücklaufsperrvertiefung (60) herausbewegt ist, wenn der mindestens eine Kupplungsfreigabevorsprung (58) aus der mindestens einen Kupplungsvertiefung (56) herausbewegt ist, und sich oberhalb oder auf der die Rücklaufsperrvertiefung (60) aufweisenden Kupplungsfläche (38) bewegt, bis der mindestens eine Kupplungsfreigabevorsprung (58) beim Weiterdrehen des Gehäuses (16) in die mindestens eine Kupplungsvertiefung (56) wieder eingetaucht ist.

2. Vorrichtung nach Anspruch 1, **dadurch gekennzeichnet, dass** die eine Kupplungsfläche (38) mehrere Kupplungsfreigabevorsprünge (58) und die andere Kupplungsfläche (36) eine der Anzahl der Kupplungsfreigabevorsprünge (58) gleichende Anzahl von Kupplungsvertiefungen (56) aufweist oder eine Anzahl von Kupplungsvertiefungen (56) aufweist, die um den Faktor 2 oder 3 oder mehr größer ist als die Anzahl der Kupplungsfreigabevorsprünge (58), wobei die relative Anordnung einer jeweils der Anzahl der Kupplungsfreigabevorsprünge (58) gleichenden Anzahl von Kupplungsvertiefungen (56) gleich der relativen Anordnung der Kupplungsfreigabevorsprünge (58) ist.

3. Vorrichtung nach Anspruch 1 oder 2, **dadurch gekennzeichnet, dass** die eine Kupplungsfläche (36) eine größere Anzahl von Rücklaufsperrvertiefungen (60) aufweist als Rücklaufsperrvorsprünge (62) an der anderen Kupplungsfläche (38) angeordnet sind.

4. Vorrichtung nach Anspruch 2 oder 3, **dadurch gekennzeichnet, dass** die Anzahl der Rücklaufsperrvertiefungen (60) gleich oder um den Faktor 2 oder 3 oder mehr größer ist als die Anzahl der Rücklaufsperrvorsprünge (62).

5. Vorrichtung nach einem der Ansprüche 1 bis 4, **dadurch gekennzeichnet, dass** die eine Kupplungsfläche (38) drei um jeweils 120° zueinander versetzt angeordnete Kupplungsfreigabevorsprünge (58) und die andere Kupplungsfläche (36) sechs um jeweils 60° zueinander versetzt angeordnete Kupplungsvertiefungen (56) aufweist und dass die eine Kupplungsfläche (38) drei um jeweils 120° zueinander versetzt angeordnete Rücklaufsperrvorsprünge (62) und die andere Kupplungsfläche (36) sechs um jeweils 60° zueinander versetzt angeordnete Rücklaufsperrvertiefungen (60) aufweist, wobei die Rücklaufsperrvertiefungen (60) jeweils mit der Mitte zwischen zwei Kupplungsvertiefungen (56) fluchtend angeordnet sind.

6. Vorrichtung nach einem der Ansprüche 1 bis 5, **dadurch gekennzeichnet, dass** der mindestens eine oder jeder Kupplungsfreigabevorsprung (58) und die mindestens eine oder jede Kupplungsfreigabevertiefung (56) auf einer konzentrisch zur Drehwelle (18) verlaufenden ersten Kreislinie angeordnet sind und dass der mindestens eine oder jeder Rücklaufsperrvorsprung (62) und die mindestens eine oder jede Rücklaufsperrvertiefung (60) auf einer konzentrisch zur Drehwelle (18) verlaufenden zweiten Kreislinie angeordnet ist, die verschieden ist von der ersten Kreislinie.

7. Vorrichtung nach einem der Ansprüche 1 bis 6, **dadurch gekennzeichnet, dass** die Drehwelle (18) in dem Gehäuse (16) mittels Lagerbuchsen (46,48,50) drehbar gelagert ist und/oder dass die Drehwelle (18) sich durch das Mitnehmerelement (32) hindurch erstreckt und/oder dass auf der Drehwelle (18) eine Schraubendruckfeder (44) konzentrisch angeordnet ist, die sich zwischen einer Stützfläche im Gehäuse (16) und einer Stützfläche an der Drehwelle (18) erstreckt und abstützt.

8. Vorrichtung nach einem der Ansprüche 1 bis 7, **dadurch gekennzeichnet, dass** der mindestens eine oder jeder Zusammengreifvorsprung (24) und der mindestens eine oder jeder Kupplungsfreigabevorsprung (58) sphärisch ausgebildet ist und dass der mindestens eine oder jeder Rücklaufsperrvorsprung (62) und die mindestens eine oder jede Rücklaufsperrvertiefung (60) rechtwinklig oder im Wesentlichen rechtwinklig zu den Kupplungsflächen (36,38) verlaufende Anlageflächen aufweisen, die aneinander liegen, wenn der mindestens eine Kupplungsfreigabevorsprung (58) in die mindestens eine Kupplungsvertiefung (56) eingetaucht ist.

## Claims

1. A device for mounting and demounting a hollow needle on and from a Phaco instrument handpiece, comprising
- a manually operable housing (16),
- a rotary shaft (18) rotatably mounted in the housing (16), the rotary shaft (18) comprising a plug-on end (22) extending outwards from an opening (20) of the housing (16) for plugging on the hollow needle (12), the plug-on end (22) comprising internal engaging projections (24) for engaging with recesses (30) in the outside of the hollow needle for the purpose of rotating the hollow needle (12) when manually rotating the housing (16),
- a torque limit and non-return coupling (34) having a contact surface (40) arranged at the rotary shaft (18) as a first coupling surface (36), and
- a driver element (32) arranged coaxially to the rotary shaft (18) in the housing, being coupled to the housing (16) and having a contact surface (42) as a second coupling surface (38) of the torque limit and non-return coupling (34),
**characterized in that**
- the two coupling surfaces (36, 38) rest against each other by being spring-loaded against each other,
- the one coupling surface (38) has at least one coupling release projection (58) and the other coupling surface (36) has at least one coupling recess (56) being complementary to the form of the coupling release projection (58),
- the one coupling surface (38) has at least one non-return projection (62) and the other coupling surface (38) has at least one non-return recess (60),
- the at least one non-return recess (60) - viewed in circumferential direction of a circular line concentrically to the axis of the rotary shaft (18) - has an extension being larger, in particular more than twice larger than the extension of the non-return projection (62) in circumferential direction of the circular line,
- the at least one non-return projection (62) rests against one of the two ends of the non-return recess (60) arranged as viewed in the circumferential direction of the circular line when the at least one coupling release projection (58) is immersed in the at least one coupling recess (56),
- the at least one non-return projection (62), when viewed in axial extension of the rotary shaft (18) , has a height which is smaller than the height of the at least one coupling release projection (58), and
- the at least one non-return projection (62) is entirely moved out of the at least one non-return recess (60) when the at least one coupling release projection (58) is moved out of the at least one coupling recess (56), and moved above or on the coupling surface (38) comprising the non-return recess (60), until the at least one coupling release projection (58) is again immersed in the at least one coupling recess (56) when the housing (16) continues to rotate.

2. The device according to claim 1, **characterized in that** the one coupling surface (38) comprises a plurality of coupling release projections (58) and the other coupling surface (36) comprises a number of coupling recesses (56) equal to the number of coupling release projections (58) or a number of coupling recesses (56) larger by factor 2 or 3 than the number of coupling release projections (58), wherein the relative arrangement of a number of coupling recesses (56) equal to the number of coupling release projections (58) is equal to the relative arrangement of the coupling release projections (58).

3. The device according to claim 1 or 2, **characterized in that** the coupling surface (36) has a larger number of non-return recesses (60) than non-return projections (62) are arranged at the other coupling surface (38).

4. The device according to claim 2 or 3, **characterized in that** the number of non-return recesses (60) is equal to or larger by factor 2 or 3 or more than the number of non-return projections (62).

5. The device according to any one of claims 1 to 4, **characterized in that** the one coupling surface (38) has three coupling release projections (58) each arranged offset by 120° relative to one another, and the other coupling surface (36) has six coupling recesses (56) each arranged offset by 60° relative to one another, and that the one coupling surface (38) has three non-return projections (62) arranged offset by 120° relative to one another, and the other coupling surface (36) has six non-return recesses arranged offset by 60° relative to one another, wherein the non-return recesses (60) are each arranged in alignment with the center between two coupling recesses (56).

6. The device according to any one of claims 1 to 5, **characterized in that** the at least one or each coupling release projection (58) and the at least one or each coupling release recess (56) are arranged on a first circular line being concentric to the rotary shaft (18), and that the at least one or each non-return projection (62) and the at least one or each non-return recess (60) is arranged on a second circular line being concentric to the rotary shaft (18) and being different from the first circular line.

7. The device according to any one of claims 1 to 6, **characterized in that** the rotary shaft (18) in the housing (6) is rotatably mounted by means of bearing bushes (46, 48, 50) and/or that the rotary shaft (18) extends through the driver element (32) and/or that a helical pressure spring (44) is arranged concentrically on the rotary shaft (18), the helical pressure spring (44) extending and being supported between a support surface in the hosing (16) and a support surface (18) on the rotary shaft.

8. The device according to any one of claims 1 to 7, **characterized in that** the at least one or each engaging projection (24) and the at least one or each coupling release projection (58) are spherically formed, and that the at least one or each non-return projection (62) and the at least one or each non-return recess (60) comprise contact surfaces extending at right angles or substantially at right angles to the coupling surfaces (36, 38) and abutting each other when the at least one coupling release projection (58) is immersed in the at least one coupling recess (56).

## Revendications

1. Dispositif de montage d'une aiguille creuse sur une pièce à main d'instrument Phaco et son démontage, doté
- d'un boîtier (16) à manipuler manuellement,
- d'un arbre rotatif (18) disposé en rotation dans le boîtier (16), lequel comporte un emmanchement (22) destiné à s'emmancher sur l'aiguille creuse (12) et s'étendant vers l'extérieur depuis une ouverture (20) du boîtier (16), dans lequel l'emmanchement (22) comporte des saillies d'engagement (24) intérieures destinées à entrer en prise mutuelle avec des évidements (30) dans la face externe de l'aiguille creuse à des fins de rotation conjointe de l'aiguille creuse (12) lors d'une rotation manuelle du boîtier (16),
- d'un accouplement limiteur de couple et antiretour (34), lequel comporte une surface d'appui (40) disposée sur l'arbre rotatif (18) en tant que première surface d'accouplement (36), et
- d'un élément d'entraînement (32) disposé dans le boîtier (16) de manière coaxiale à l'arbre rotatif (18), lequel est couplé au boîtier (16) et comporte une surface d'appui (42) comme deuxième surface d'accouplement (38) de l'accouplement limiteur de couple et antiretour (34), **caractérisé en ce que**
- les deux surfaces d'accouplement (36, 38) appuient l'une sur l'autre de manière élastique et précontrainte l'une par rapport à l'autre,
- l'une surface d'accouplement (38) comporte au moins une saillie de dégagement d'accouplement (58) et l'autre surface d'accouplement (36) comporte au moins une dépression d'accouplement (56) de forme complémentaire à celle de la saillie de dégagement d'accouplement (58),
- l'une surface d'accouplement (38) comporte au moins une saillie antiretour (62) et l'autre surface d'accouplement (38) comporte au moins une dépression antiretour (60),
- l'au moins une dépression antiretour (60) - vu dans la direction du pourtour d'une circonférence concentrique par rapport à l'axe de l'arbre rotatif (18) - comporte une étendue plus élevée que l'étendue de la saillie antiretour (62) dans la direction du pourtour de la circonférence, en particulier au moins plus du double de celle-ci,
- l'au moins une saillie antiretour (62) appuie sur une des deux extrémités de la dépression antiretour (60), vu dans la direction du pourtour de la circonférence, lorsque l'au moins une saillie de dégagement d'accouplement (58) est plongée dans l'au moins une dépression d'accouplement (56),
- l'au moins une saillie antiretour (62) comporte, vu dans l'étendue axiale de l'arbre rotatif (18), une hauteur inférieure à la hauteur de l'au moins une saillie de dégagement d'accouplement (58),
- l'au moins une saillie antiretour (62) est entièrement enlevée de l'au moins une dépression antiretour (60) lorsque l'au moins une saillie de dégagement d'accouplement (58) est enlevée de l'au moins une dépression d'accouplement (56), et se déplace au-dessus de ou sur la surface d'accouplement (38) comportant la dépression antiretour (60) jusqu'à ce que l'au moins une saillie de dégagement d'accouplement (58) soit de nouveau plongée dans l'au moins une dépression d'accouplement (56) en cas de rotation ultérieure du boîtier (16).

2. Dispositif selon la revendication 1, **caractérisé en ce que** l'une surface d'accouplement (38) comporte plusieurs saillies de dégagement d'accouplement (58) et l'autre surface d'accouplement (36) comporte un nombre de dépressions d'accouplement (56) égal au nombre de saillies de dégagement d'accouplement (58) ou comporte un nombre de dépressions d'accouplement (56) supérieur d'un facteur 2 ou 3 ou plus au nombre de saillies de dégagement d'accouplement (58), dans lequel la disposition relative d'un nombre de dépressions d'accouplement (56) respectivement égal au nombre de saillies de dégagement d'accouplement (58) est la même que la disposition relative des saillies de dégagement d'accouplement (58).

3. Dispositif selon la revendication 1 ou 2, **caractérisé en ce que** l'une surface d'accouplement (36) comporte plus de dépressions antiretour (60) qu'il n'y a de saillies antiretour (62) sur l'autre surface d'accouplement (38).

4. Dispositif selon la revendication 2 ou 3, **caractérisé en ce que** le nombre de dépressions antiretour (60) est égal ou supérieur d'un facteur 2 ou 3 ou plus au nombre de saillies antiretour (62).

5. Dispositif selon l'une des revendications 1 à 4, **caractérisé en ce que** l'une surface d'accouplement (38) comporte trois saillies de dégagement d'accouplement (58) disposées en décalage de 120° l'une par rapport à l'autre et l'autre surface d'accouplement (36) comporte six dépressions d'accouplement (56) disposées en décalage de 60° l'une par rapport à l'autre et **en ce que** l'une surface d'accouplement (38) comporte trois saillies antiretour (62) disposées en décalage de 120° l'une par rapport à l'autre et l'autre surface d'accouplement (36) comporte six dépressions antiretour (60) disposées en décalage de 60° l'une par rapport à l'autre, dans lequel les dépressions antiretour (60) sont disposées en alignement avec leur milieu respectivement entre deux dépressions d'accouplement (56).

6. Dispositif selon l'une des revendications 1 à 5, **caractérisé en ce que** l'au moins une ou chaque saillie de dégagement d'accouplement (58) et l'au moins une ou chaque dépression de dégagement d'accouplement (56) sont disposées sur une première circonférence concentrique par rapport à l'arbre rotatif (18) et **en ce que** l'au moins une ou chaque saillie antiretour (62) et l'au moins une ou chaque dépression antiretour (60) sont disposées sur une deuxième circonférence concentrique par rapport à l'arbre rotatif (18) et différente de la première circonférence.

7. Dispositif selon l'une des revendications 1 à 6, **caractérisé en ce que** l'arbre rotatif (18) est disposé en rotation dans le boîtier (16) au moyen de douilles de palier (46, 48, 50) et/ou **en ce que** l'arbre rotatif (18) s'étend à travers l'élément d'entraînement (32) et/ou **en ce qu'**est disposé de manière concentrique sur l'arbre rotatif (18) un ressort de compression hélicoïdal (44), lequel s'étend entre et soutient une surface de soutien dans le boîtier (16) et une surface de soutien sur l'arbre rotatif (18).

8. Dispositif selon l'une des revendications 1 à 7, **caractérisé en ce que** l'au moins une ou chaque saillie d'engagement (24) et l'au moins une ou chaque saillie de dégagement d'accouplement (58) sont réalisées de manière sphérique et **en ce que** l'au moins une ou chaque saillie antiretour (62) et l'au moins une ou chaque dépression antiretour (60) comportent des surfaces d'appui perpendiculaires ou sensiblement perpendiculaires aux surfaces d'accouplement (36, 38), lesquelles reposent l'une sur l'autre lorsque l'au moins une saillie de dégagement d'accouplement (58) est plongée dans l'au moins une dépression d'accouplement (56).
